# EUROPEAN PATENT APPLICATION

(11) **EP 3 561 038 A1**
(43) Date of publication of application: **30.10.2019**
(21) Application number: 18169131.2
(22) Date of filing: 24.04.2018
(51) Int. Cl.: C12M 1/00

(54) **CELL SEPARATOR**

(71) Applicant: Bayer AG, 51373 Leverkusen (DE)
(72) Inventor: Jenne, Marc, 51375 Leverkusen (DE); Brod, Helmut, 51061 Köln (DE); Kauling, Joerg, 51427 Bergisch Gladbach (DE); Möbus, Helge, 97702 Münnerstadt (DE)
(74) Representative: BIP Patents

(57) **Abstract**

What is described herein relates to a prechamber for an inclined channel gravity cell separator with a multitude of channels for retaining cells from a reactor mixture, wherein said prechamber comprises at least one inlet for flow distribution of an incoming fluid stream and at least one aperture towards the multitude of channels and wherein said prechamber encloses a 3-dimensional space.

## Description

Usually recombinant proteins such as monoclonal antibodies are produced in mammalian cells, since these cells are capable of correctly processing the recombinant proteins. In general batch, fed batch or perfusion cell cultures are used. As higher cell densities can be reached the perfusion process typically employs considerably smaller bioreactors. To achieve high cell densities (> 20 million live cells per milliliter) in a continuously operated bioreactor, efficient retention of the cells is necessary. The required degree of retention depends in this case on the growth rate of the cells and on the perfusion rate q/V (media throughput q per bioreactor volume V). The necessary selective retention of viable cells can for example be performed by inclined-channel gravity cell settlers.

Compared with simple settling tanks inclined channel separators on large scales have the advantage of considerably lower volume in relation to the separation area. A publication (Henzler, H.-J., Chemie-Technik, 1, 1992, 3) describes cell retention in inclined channel separators, which can be operated in counterflow, crossflow and in co-current flow. The flow-bearing channel cross section can be provided with plates or tubes. Usually, the inclined channel separators are arranged outside the bioreactor and attached to the bioreactor via an external circuit in order to ensure an easy accessibility of the cell retention system for maintenance and cleaning purposes.

Overall, high cell densities and an associated high productivity in continuous bioreactors may be achieved, if the following requirements are met:
- an adequate and low-shear supply of the cells with substrates, in particular dissolved oxygen,
- an adequate disposal of the carbon dioxide formed in respiration,
- an adequate disposal of cell debris and dead cells.
- an effective, low-shear, sustainable cell retention system for building up high cell densities.

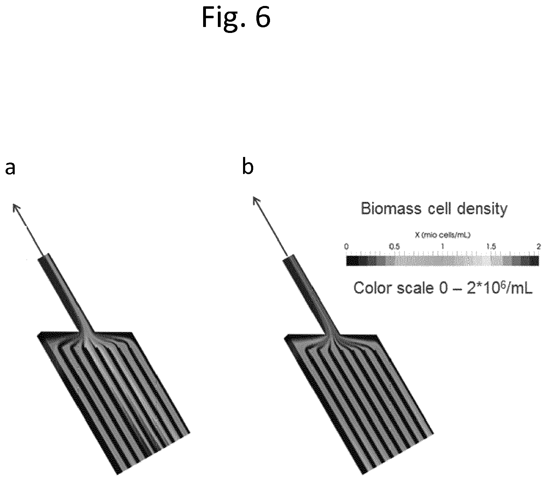

Moreover, to minimize cell damage, in particular owing to inadequate oxygen supply and carbon dioxide removal outside the bioreactor, and also degradation of active ingredients owing to enzymatic activity, cell retention systems with short residence times of the cells in the cell retention system are ideal. One way of minimizing the residence time of cells in the cell retention system is to increase the flow rate in the system. However, this can lead to a wash out of cells at the top of the channels of an inclined channel gravity cell separator (cf. FIG 6a). This wash out in turn reduces the overall efficiency of the system.

Thus there is the need for a device and methods for increasing the efficiency of cell settlers.

This objective is solved by a prechamber for an inclined channel gravity cell separator with a multitude of channels for retaining cells from a reactor mixture, wherein said prechamber comprises at least one inlet for flow distribution of an incoming fluid stream and at least one aperture towards the multitude of channels and wherein said prechamber encloses a 3-dimensional space.

This prechamber has the advantage that it leads to a more even and ideally equal distribution of the incoming fluid stream into the channels of the inclined channel gravity cell separator. Thus, the cell load imposed on individual channels is more equally distributed to the channels, resulting in a higher overall efficiency of the inclined channel gravity cell separator. This is the case as cell loss at the top of the channels due to overload of individual channels is minimized.

In addition, the prechamber described herein facilitates minimized resuspension of the already sedimented cells and reintroduction into the channels due to back-mixing with the incoming fluid stream (feed flow).

Moreover possible obstructions to the flow of the reactor mixture are minimized.

As used herein the term "inclined channel gravity cell separator" refers to a device for the retention of cells e.g. from a bioreactor mixture comprising channels which open at the lower and the upper region and are inclined against the direction of gravity i.e. tilted during operation.

Typically, at their lower end the channels lead to at least one receiving chamber comprising a collection region and at their upper end the cell depleted reactor mixture (process fluid, e.g. the harvest) is withdrawn from the inclined channel gravity cell separator.

Thus, cells and cell culture solution are separated in the channels of the inclined channel gravity cell separator. This happens for example, if the cell depleted reactor mixture (process fluid, e.g. the harvest), i.e. the clarified reactor mixture is removed from the upper end of the channels. As a result of this removal reactor mixture comprising the cells to be separated, e.g. cell culture solution from a bioreactor, is sucked into the channels at their lower end. The cells sediment in the channels and counterslide on the underside of the channels against the flow direction to the lower channel ends. Thus cells sediment within the tilted channels and slide in countercurrent flow with respect to the inflowing reactor mixture out of the channels and are collected in the collection region of the at least one receiving chamber.

As used herein the term "prechamber" refers to a device which minimizes back mixing of the separated cells that slide down the channels due to gravity and the inflowing reactor mixture comprising more cells to be separated and which at the same time enables a more even and ideally equal distribution of the flow of the reactor mixture into the channels. The prechamber encloses a three dimensional (3 D) space, i.e. it can hold a predetermined volume of e.g. the incoming fluid stream.

In some embodiments the incoming fluid stream is a reactor mixture.

As used herein the term "reactor mixture" refers to the process fluid comprising the cells to be settled and thereby separated from the fluid they are supplied in.

As used herein the term "aperture" refers to a part of the prechamber through which the incoming fluid stream, i.e. the reactor mixture enters the channels of the inclined channel gravity cell separator. Thus, the aperture facilitates the equal distribution of incoming reactor mixture into the multitude of channels.

In some embodiments the prechamber is formed by an insert and at least one wall of the inclined channel gravity cell separator. In some of these embodiments least one opening is provided opposite to the at least one aperture at the lower end of the prechamber.

As used herein the term "insert" refers to a device that can be introduced into an inclined channel gravity cell separator e.g. into at least one receiving chamber of the inclined channel gravity cell separator.

The at least one opening opposite to the at least one aperture typically leads to a collection region of the at least one receiving chamber of the inclined channel gravity cell separator. It allows the transfer of portions of incoming fluid stream enriched with cells to the receiving chamber and thus prevents the formation of stagnant, non-moved zones with insufficient supply of oxygen to the cells in the prechamber. Stagnant, non-moved zones with insufficient supply of oxygen can cause cell death and finally the release of unwanted impurities, e.g. host cell proteins (HCP's).

In theory when considering flow conditions that prevent cell sedimentation in the prechamber, it is possible to operate the prechamber without the at least one opening opposite to the at least one aperture. However, as the exact composition and the flow conditions of the reactor mixture can vary during operation the prechamber described herein preferably comprises at least one opening opposite to the at least one aperture.

In addition, the at least one opening of the prechamber opposite to the at least one aperture allows larger and smaller cell aggregates to directly enter the receiving chamber.

As mentioned above, in some embodiments the insert is positioned in a receiving chamber of the inclined channel gravity cell separator and hence the prechamber is formed by at least one wall of the receiving chamber and the insert. In some cases of these embodiments, the at least one inlet for flow distribution of an incoming fluid stream is an inlet of the receiving chamber, which was already present before the insert was introduced into the receiving chamber to form the prechamber.

In some embodiments, the insert is spatially fixed via jamming it into the receiving chamber, via guiding it i.e. the as insert is led in grooves, or screwed in. These embodiments enable removal of the insert from the receiving chamber e.g. for cleaning purposes.

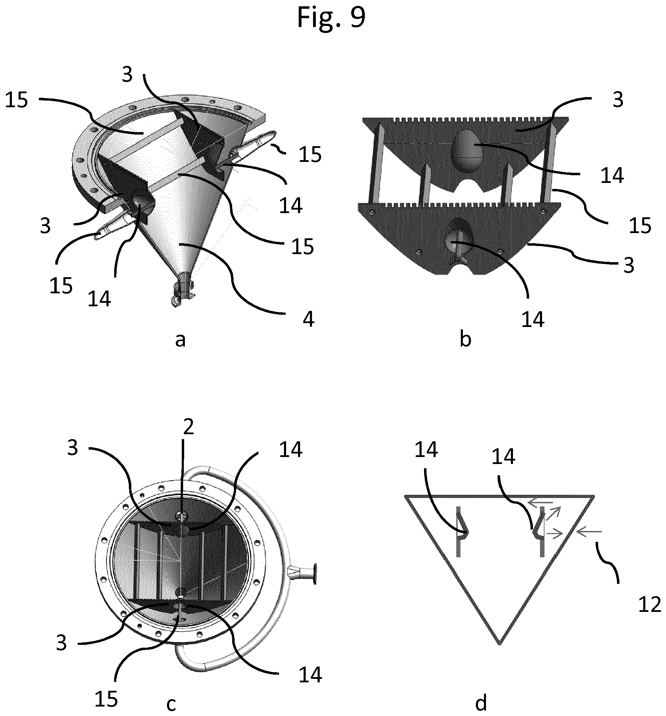

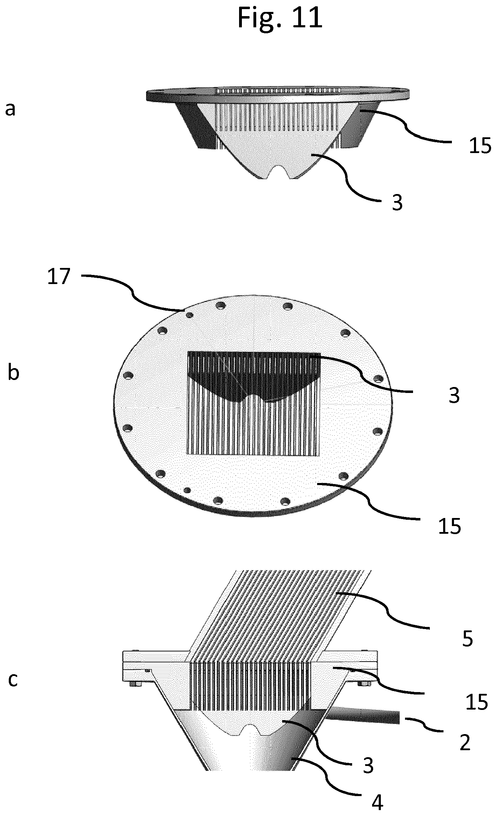

In some embodiments the insert is spatially fixed using anti-rotational devices e.g. those depicted in Fig. 9-11. In the case of the embodiments schematically illustrated in Fig. 11 each aperture of the insert is a longitudinal opening which is horizontally parallel to a lower part of the multitude of channels. Thus, the apertures of the insert in this example together with the multitude of channels of the inclined channel gravity cell separator form a comb-like structure, since exactly one aperture is positioned at every channel of the multitude of channels.

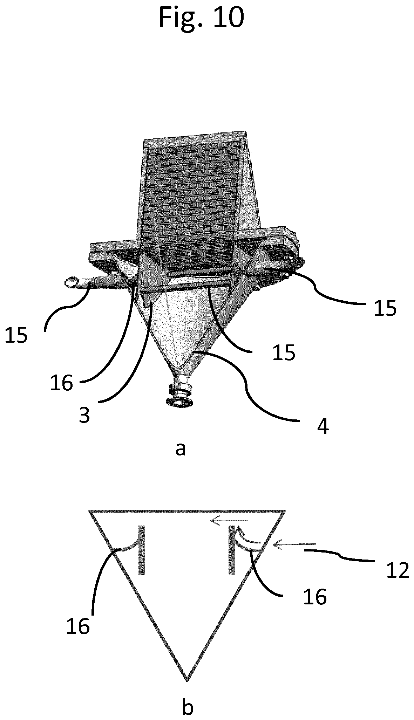

In case of embodiments schematically depicted in Fig. 9 and Fig. 10 the anti-rotational devices are designed as cylindrical elements that are introduced into the at least one inlet for flow distribution of an incoming fluid stream. In some embodiments these cylindrical elements are designed to realize minimum wall contact within the inlet Fig.9).

In some embodiments the antirotational device provides an inclined surface in order to upwardly deflect the inflowing reactor mixture to the at least one aperture (cf. Fig. 10)

The minimum area of the at least one aperture of the prechamber is designed in a way that the overpressure in the prechamber does not exceed the hydrostatic pressure difference between sediment in the receiving chamber and the reactor mixture in the prechamber. This prevents the unwanted short cut of reactor mixture into the sediment of the receiving chamber via the at least one opening opposite to the at least one aperture. The width of the indentation is designed in a way that particles are not captured to prevent a slowly blocking of the at least one aperture during the processing of the reactor mixture.

In some embodiments the inclined channel gravity cell separator is autoclavable.

In some embodiments the channels of the inclined channel gravity cell separator have a square cross section. However, other channel geometries such as round, rectangular or eclipse shapes are possible.

In some embodiment the inclined channel gravity cell separator is characterized by:
- A theoretical settler area of 1,5 -20 m², preferably 2 - 3, most preferably 2,4 m² and/or
- a reactor mixture flow (settler feed-flow) of 10-4000 L/h, preferably of 100-200 L/h, most preferably of 160 L/h and/or
- a plate number of 10-100, preferably 15-25, most preferably of 25, and/or
- a plate length of 0.3-2 m, preferably of 0,5-1,5 m, most preferably of 1 m, and/or
- a plate distance of 3-10 mm , preferably 4-6 mm, most preferably 5 mm

In some embodiments the insert in characterized by :
- an indentation number which equals the number of plates + 1 and/or
- an indentation with of 1-6 mm, preferably 2-4 mm most preferably 3 mm and/or
- an indentation height of 10 - 200 mm, preferably of 25-35mm, most preferably of 30 mm

Alternatively, the prechamber designed as insert can be welded and/or fixed via adhesive bonding.

Moreover to a person skilled in the art it is apparent that the insert can be spatially fixed within the receiving chamber of an inclined channel gravity cell separator using a combination of the above described means or in case that a receiving chamber comprises more than one insert, the different inserts can be fixed by different means.

In some embodiments the at least one prechamber comprises as only one aperture.

In some embodiments the at least one prechamber comprises several apertures. In some of these embodiments, where the at least one prechamber comprises several apertures these apertures are all of the same dimensions. In alternative embodiments where the at least one prechamber comprises several apertures at least two apertures of a given prechamber differ in their dimensions. These differences in dimensions can reflect the difference in flow velocity of the incoming reaction mixtures, e.g. channels where a high flow velocity is expected have smaller apertures to redirect more of the incoming reaction mixture to other channels with larger apertures.

In some embodiments the at least one prechamber comprises as many apertures as there are channels in the inclined channel gravity cell separator.

In some embodiments of the prechamber described above each of said at least one apertures consist of at least one indentation, which is open and at least one elevation, which is closed.

In some embodiments of the prechamber described above for each of said at least one apertures the at least one indentation is a square or a concave cross section to the receiving chamber employing an increased indentation height from the center towards the both walls of the inclined channel where this increase is defined by the pressure or flow profile in the prechamber.

As used herein the term "indentation" refers to the part of the aperture which is open. In other words the reactor mixture enters the channels of the inclined channel gravity cell separator via the at least one indentation of the at least one aperture.

As used herein the term "elevation" refers to the part of the aperture which is closed and thus minimizes back mixing of the separated cells that slide down the channels due to gravity and inflowing reactor mixture comprising more cells to be separated.

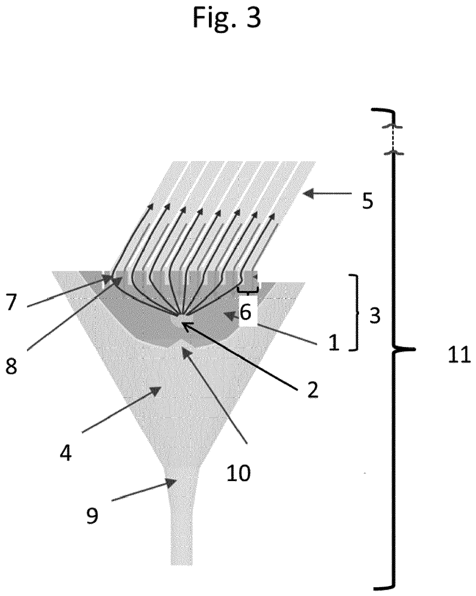

Thus, the reactor mixture enters the inclined channel gravity cell separator via the apertures of the prechamber, which equally distribute the reactor mixture, while the combination of indentation and elevations of said apertures minimizes back mixing of the inflowing reactor mixture with the separated cells (cf. FIG 3).

In some embodiments each of the at least one aperture consist of exactly one indentation, which is open and exactly one elevation, which is closed.

In some embodiments of the prechamber described above said prechamber is positioned outside of the inclined channel gravity cell separator and said prechamber either alone or in combination with at least one wall of the inclined channel gravity cell separator forms the at least one aperture.

In some embodiments of the prechamber positioned outside of the inclined channel gravity cell separator, the prechamber further comprises at least one opening wherein the at least one opening connects the prechamber with a receiving chamber of the inclined channel gravity cell separator.

In some embodiments, the insert and/or the prechamber comprise additional means to guide the incoming reactor mixture such as dimples (14) and spoons (15) schematically illustrated in Fig. 9 and Fig. 10.

In some embodiments the prechamber described herein is made of disposable material.

In another aspect what is described herein relates to an inclined channel gravity cell separator with a multitude of channels for retaining cells from a reactor mixture comprising
- at least one receiving chamber comprising at least one collection region for collecting the cells using gravity,
- wherein the receiving chamber has an outlet at the bottom allowing the retained cells to exit the inclined channel gravity cell separator and wherein the receiving chamber is in fluid communication with the multitude of channels which are opened at their lower end and at their upper end and
- wherein the inclined channel gravity cell separator further comprises at least one prechamber as described above, wherein the at least one aperture of the at least one prechamber is in direct fluid communication with the channels of the inclined channel gravity cell separator.

During an exemplary mode of operation the reactor mixture comprising cells to be separated arrives at a prechamber of an inclined channel gravity cell separator and enters the prechamber via an inlet of the prechamber. In the prechamber the reactor mixture is distributed upwards into the multitude of channels of the inclined channel gravity cell separator via the apertures of the prechamber. The reactor mixture can only pass the apertures at the indentations, which are open. This upward distribution is a result of a removal of the reactor mixture from the inclined channel gravity cell separator at the upper end of the channels. Due to the interaction of factors such as gravity and flow velocity of the reactor mixture as well as cell density etc. of the reactor mixture the cells are separated from the lighter contents of the reactor mixture in the tilted channels. The lighter contents with low particle density and low sedimentation velocity are washed out. The clarified reactor mixture (process fluid e.g. the harvest) leaves the channels at their upper end whereas the cells are caused by gravity to slide down the channels on the channel underside and exit the channels at their lower end towards the collection region of the receiving chamber. Due to the elevation of the apertures of the prechamber and the fact that the cells counterslide on the underside of the channels, the separated cells do not mix with the incoming reactor mixture - i.e. the reactor mixture enters the individual channels at the channel wall opposite the wall at which the cells slide down and the closed elevations minimize any back mixing. Instead the cells that were separated from the reactor mixture in the channels enter the collection region of the receiving chamber. The cells leave the receiving chamber via an outlet e.g. located at the bottom of the receiving chamber for example to be returned to the bioreactor or to be discarded.

In some embodiments of the inclined channel gravity cell separator described herein the inclined channel gravity cell separator comprises one receiving chamber with two prechambers, wherein each prechamber comprises one inlet and wherein said prechambers are formed by an insert and at least one wall of the inclined channel gravity cell separator and wherein at least one opening is present opposite to the at least one aperture of each prechamber.

Preferably, the two inlets for flow distribution of the reactor mixture are located exactly opposite to each other.

Another aspect what is described herein relates to the use of a prechamber as described above or an inclined channel gravity cell separator as described above, for cell separation.

In yet another aspect what is described herein relates to a method of separating cells from a reactor mixture and retaining the cells comprising the following steps:
- providing a reactor mixture, comprising cells to be separated, to an inclined channel gravity cell separator as described above comprising at least one prechamber via at least one inlet
- flow distribution of the reactor mixture into the channels of the inclined channel gravity cell separator via the at least one aperture of the at least one prechamber
- separation of the cells from the reactor mixture in the channels of the inclined channel gravity cell separator wherein the clarified reactor mixture exits the channels at their top end and the separated cells slide down the channel wall,
- collection of the separated cells in the receiving chamber of the inclined channel gravity cell separator.

Furthermore, what is described herein relates to an insert for generating a prechamber according to what is described above, wherein said insert has a body allowing for the formation of at least one aperture towards the multitude of channels as well as for the formation of the at least one opening opposite to the at least one aperture, if the insert is positioned in the inclined channel gravity cell separator.

An example of such an insert for generating a prechamber is a baffle plate.

### Example

In this example the inclined channel gravity cell separator is autoclavable and has a settler area of 2,4 m². Its channels have a square cross section. Moreover, the inclined channel gravity cell separator has a reactor mixture flow (settler feed-flow) of 160 L/h, 25 plates with a length of 1m and a plate distance of 5mm. This exemplary inclined channel gravity cell separator has two inserts, each with 26 indentations, each of the indentations is 3 mm wide and 30 mm high.

### Figures

### List of reference signs:

- prechamber (1)
- inlet (2)
- insert (3)
- receiving chamber (4)
- a multitude of channels (5)
- aperture (6)
- indentation (7)
- elevation (8)
- collection region (9)
- opening (10)
- inclined channel gravity cell separator (11)
- reactor mixture flow (12)
- separated cells sliding down channel wall (13)
- dimple (14)
- anti-rotation device (15)
- spoon (16)

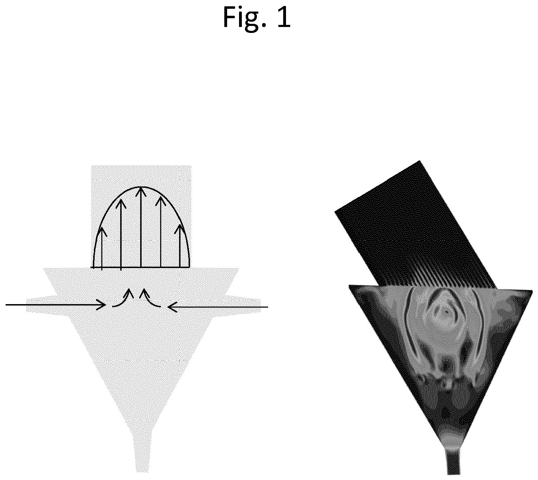

   Fig. 1 (left: front view, right: 90° rotated side view) is a schematic illustration of a simulation of the typical flow distribution of a reactor mixture in an inclined channel gravity cell separator with a multitude of channels for retaining cells. It can be clearly seen that if the reactor mixture arrives evenly distributed to both opposite inlets of the receiving chamber of the inclined channel gravity cell separator with a multitude of channels usually increased velocity patterns due to stagnation flow are observed in the center of the receiving chamber, which finally result in a higher cell load to the central parts of the channels compared to those channel parts nearest to the inlets. This can lead to an increased wash out of the cells at the top end of the channels due to a cell breakthrough via the center region of the channels. These cells which are washed out are lost and hence decrease the overall efficiency of the system.

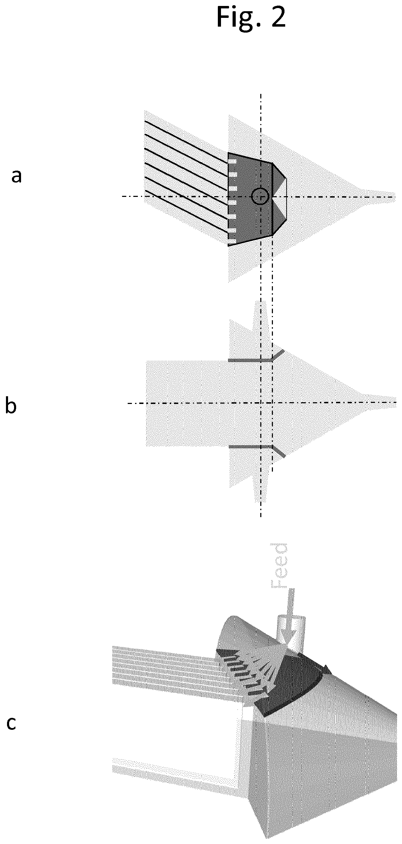

   Fig. 2 is a schematic illustration of part of an inclined channel gravity cell separator (11) with a multitude of channels (5) for retaining cells. Shown are part of a receiving chamber (4) and the lower part of the multitude of channels (5). Here the inclined channel gravity cell separator (11) comprises two prechambers (1) as described herein. In this example the prechambers (1) are formed by an insert (3) and at least one wall of the inclined channel gravity cell separator (11) and at least one opening (10) is present opposite to the at least one aperture (6). The prechambers (1) are positioned to receive the reactor mixture flow (12) of the two opposite inlets (2) into the receiving chamber (4). Thus, when the two inserts (3) are positioned in the receiving chamber (4) between each of them and at least one wall of the receiving chamber (4) of the inclined channel gravity cell separator (11) an opening (10) is formed. Opposite to said opening (10) formed with the wall of the receiving chamber (4) of the inclined channel gravity cell separator (11) at least one - here several - apertures (6) are positioned wherein each of said apertures (6) consist of at least one indentation (7), which is open, and at least one elevation (8), which is closed.
      In other words, in a 3D-view FIG 2c shows how reactor mixture (12) entering the receiving chamber of the inclined channel gravity cell separator (11) via one of the two opposite inlets (2) is distributed by one of the two prechambers (1) into the multitude of channels (5) of the inclined channel gravity cell separator (11). Moreover, the insert (3) is narrow compared to the complete channel width. Thus, the space for the separated cells to vertically enter the receiving chamber (4) along the direction of gravity is not reduced by the prechambers .
   FIG 2b shows the side view of the prechamber (1) with the feed inlets (2) to both sides of the wall of the receiving chamber (4).
   FIG 2a shows front the view of the prechamber (1) of FIG 2b turned by 90° counterclockwise, i.e. the viewed prechamber insert is depicted from the side of the inlet (2).
   FIG. 3 represents a larger schematic illustration of FIG. 2a. Depicted are a part of an inclined channel gravity cell separator (11) as described herein, comprising an inlet (2) for flow distribution, a receiving chamber (4) a multitude of channels (5), a collection region (9) of the receiving chamber (4) and a prechamber (1) formed by an insert (3) comprising apertures (6) with indentations (7), and elevations (8) and an opening opposite (10) to the at least one aperture (6).
      During an exemplary mode of operation reactor mixture (12) comprising cells to be separated arrives at the prechamber (1) via an inlet (2) for flow distribution. The main part of the reactor mixture (12) is distributed upwards into the multitude of channels (5) of the inclined channel gravity cell separator via the apertures (6) of the prechamber (1), which is formed in this case by the insert (3) and the wall of the receiving chamber (4). The reactor mixture can only pass the apertures (6) at the indentations (7), which are open, but not via an elevation (8). The upward distribution is a result of a removal of the reactor mixture (12) from the inclined channel gravity cell separator (11) at the upper end of the channels (not shown). The prechamber (1) has an opening (10) opposite to the at least one aperture (6), which is here formed between at least one wall of the receiving chamber (4) of the inclined channel gravity cell separator (11) and the insert (3).
      Depending on the interaction of factors such as gravity and flow velocity of the reactor mixture (12) as well as cell density etc. of the reactor mixture (12) the cells are separated from the lighter contents of the reactor mixture such as fermentation media, dead cells and cell debris in the tilted channels (5). The clarified reactor mixture leaves the channels at their upper end (not shown) whereas the cells are caused by gravity to slide down the channels (13) and exit the channels (5) at their lower end towards the collection region (9) of the receiving chamber (4). Due to the elevation (8) of the apertures (6) of the insert (3) and the fact that the cells counterslide on the underside of the channels (5), the separated cells should not mix with the incoming reactor mixture - i.e. the reactor mixture (12) enters the individual channels at the channel wall opposite to the wall at which the cells slide down (13) with the closed elevations (8) hindering the entrance to the zones of separated cells (13) and thus minimizing back mixing. Instead the cells that were separated (13) from the reactor mixture (12) in the multitude of channels (5) enter the collection region (9) of the receiving chamber (4). The cells leave the receiving chamber (4) via an outlet e.g. located at the bottom of the receiving chamber for example to be returned to the bioreactor or to be discarded.

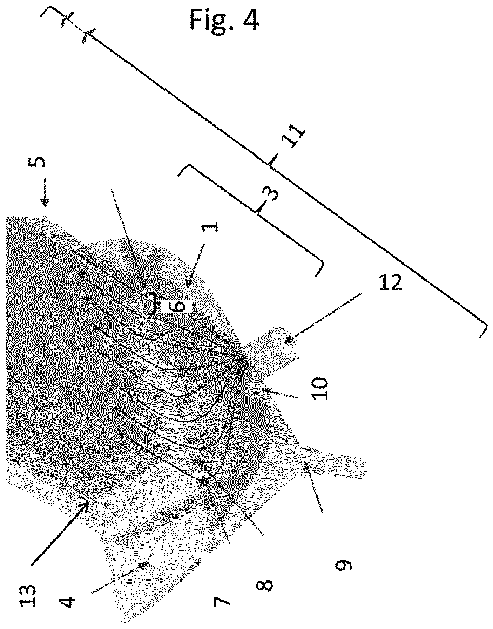

   FIG. 4 depicts the same parts of an inclined channel gravity cell separator as shown in FIG. 3 but from a different perspective. In this perspective it can be seen, that due to the prechamber (1) the separated cells (13) mainly slight down in the region behind the elevation (8) and thus do not interact with the reaction mixture (12), which is inflowing by the indentation (7) under minimizing back mixing of both flows (12) and (13).

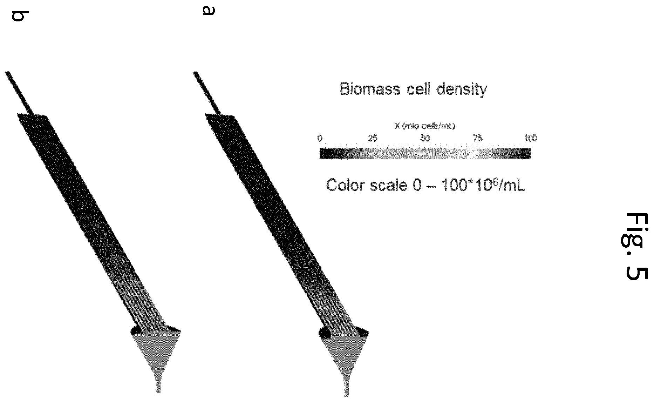

   Fig. 5 illustrates the spatial distribution of cell density X inside a pilot scale inclined channel gravity cell separator as a whole. It can be clearly seen that the biomass hold up in the channel section of the pilot scale inclined channel gravity cell separator is higher without any prechamber. In the case without any prechamber, the biomass hold up is 45.1 billion cells (a). In the case with a prechamber inside the pilot scale gravity cell separator, the biomass hold up is 37.5 billion cells (b). In this case less cells are exposed to a non-ideal environment, what is desirable and clearly shows the advantage of using a prechamber.
   Fig. 6 shows the spatial distribution of cell density X at the upper end of the channel section (5) and at the harvest outlet. In the case (a) without a prechamber, there is a higher harvest cell density X_{H} (0.375*10⁶/ml) at the harvest outlet, i.e. more cells are lost compared to (b) (0.27*10⁶/ml). As the filter positioned downstream of the harvest outlet would be blocked earlier in this case, this is not desirable and again shows the advantage of using a prechamber (1)

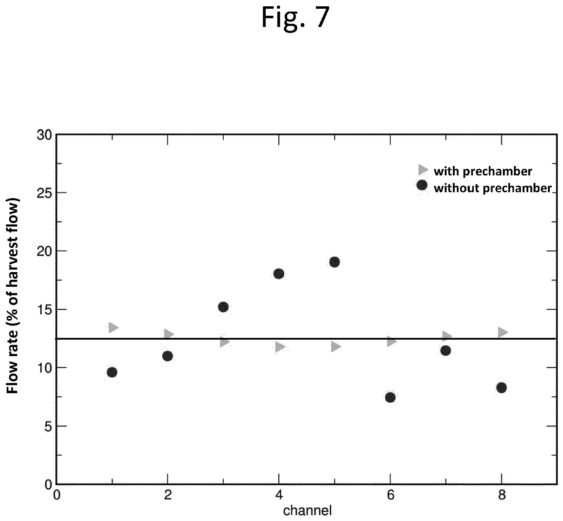

   Fig. 7 illustrates flow rate maldistribution inside the channel section for the case with prechamber (circles) and without prechamber (triangles). In case of the absence of maldistribution a flow rate being equal to harvest flow rate is equally distributed over the channels and is 1/8 of harvest flow rate (see horizontal line). The case without prechamber deviates from the horizontal line and shows an increased thoughput via the center plates. The case with prechamber almost coincides with the horizontal line.

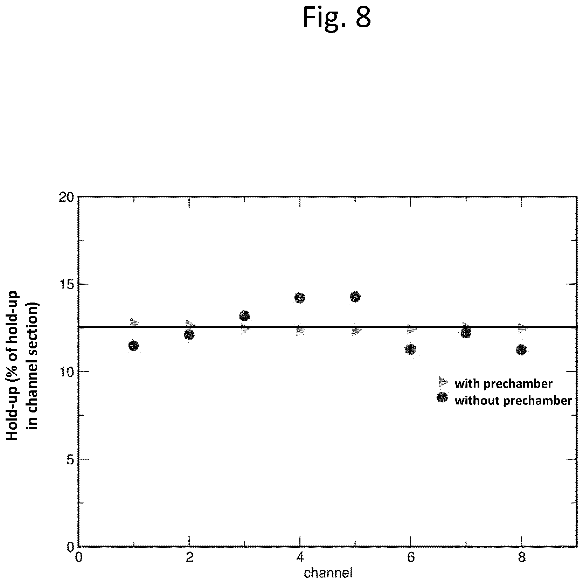

   Fig. 8 illustrates biomass (or cell) hold up maldistribution inside the channel section for the case with prechamber and without prechamber. In case of the absence of maldistribution biomass (or cell) hold up is equally distributed over the channels and is 1/8 of the whole hold up in the channel section (see horizontal line). Again, the case without prechamber deviates from the horizontal line, but not as much as in the case of flow rate maldistribution. The case with prechamber almost coincides with the horizontal line.
   Fig. 9 a-d illustrates an example of how in embodiments with an insert, said insert is spatially fixed within the at least one receiving chamber (4). In this case each of the two inserts (3) is spatially fixed with one anti-rotation device positioned within each of the two inlets (2). In addition, Fig. 9 also schematically depicts an example of inserts (3) comprising dimples (14) for guidance of the reactor mixture flow (12). As illustrated in Fig. 9 (d) the presence of the dimples (14) distributes the incoming reactor mixture flow (12) backwards and upwards to minimize a deflection of the incoming reactor mixture (12) towards the opening (10)
   Fig. 10 a & b illustrate another example of an embodiment with an insert, said insert is spatially fixed within the at least one receiving chamber (4). In this case the anti-rotational device (15) is formed as "spoon" (16). This spoon (16) in combination with additional anti rotational devices (15) spatially fixes the insert (3) in the inlet openings (2) of the receiving chamber (4). In addition, as illustrated in Fig. 10 (b) the presence of the spoons (16) distributes the incoming reactor mixture flow (12) upwards to minimize a deflection of the incoming reactor mixture (12) towards the opening (10).
   Fig. 11 a & b illustrate another example of an embodiments with an insert, said insert is spatially fixed within the at least one receiving chamber (4). In this example the insert (3) is fixed with the anti-rotational device here an intermediate flange, which is fixed with several screws e.g. screw (17) and sealed by sanitary designed O-ring gaskets vs. the flanges of the inclined channel gravity settler (11). Moreover, the apertures (6) of the insert (3) in this example comprise indentations (7) and elevations (8) and together with the multitude of channels (5) form a comb-like structure, since exactly one aperture is positioned at every channel of the multitude of channels (5). This comb like structure has the effect that the incoming reactor mixture can only pass the apertures (6) at the indentations (7), which are open, but not via an elevation (8). As described above the reactor mixture enters the individual channels at the channel wall opposite the wall at which the cells slide down and the closed elevations (8) minimize any back mixing.

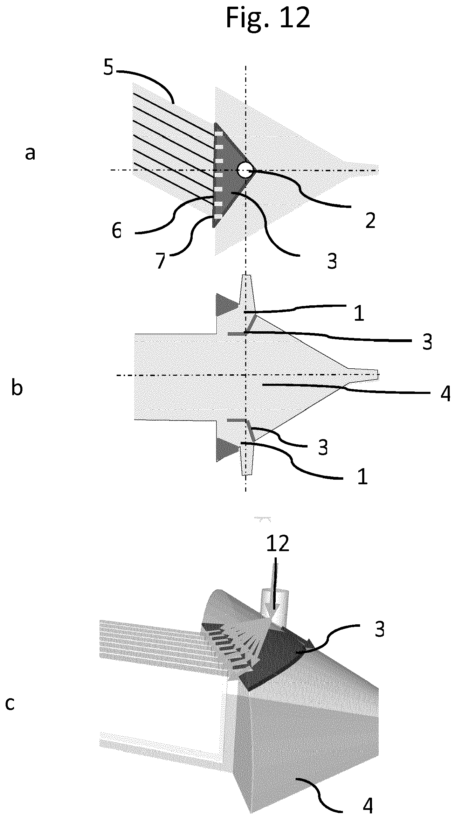

   Fig. 12c is a schematic 3D illustration of an inclined channel gravity cell separator comprising two prechambers (1) as described herein. In this example the prechambers (1) are formed by an insert (3) and at least one wall of the inclined channel gravity cell separator (10). However, this example differs from the exemplary inclined channel gravity cell separator depicted in Fig. 2 as no opening is present opposite to the at least one aperture (6). In these embodiments the flow rate through out the entire separation is high enough to prevent sedimentation of cells of a reactor mixture (12) within the prechamber to allow a fully transfer reactor mixture (12) to the receiving chamber of the inclined channel gravity cell separator (11) via the at least aperture (6) of the inset (3)
   FIG 12b shows the side view of the prechamber (1) with the inlet ports (2) to both sides of the receiving chamber (4)
   FIG 12a shows the front view of the prechamber (1) of FIG 12b turned by 90° counterclockwise, i.e. the viewed prechamber insert is depicted from the side of the inlet (2).

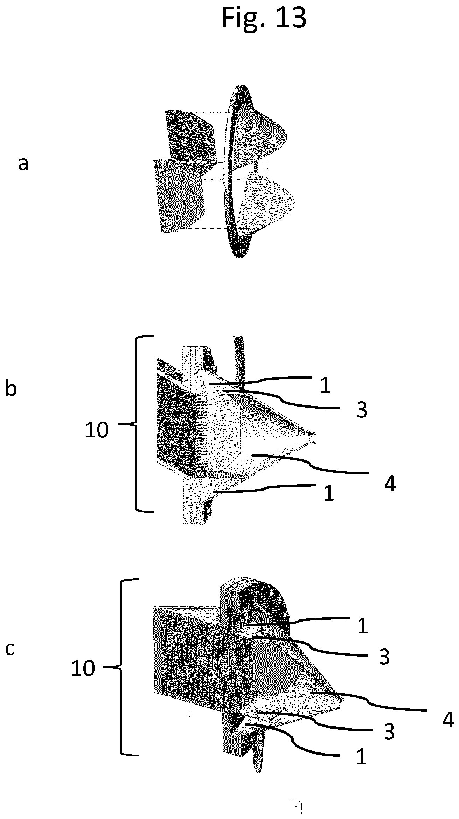

   Fig. 13a-c are schematic 3D illustrations of an inclined channel gravity cell separator comprising two prechambers (1) as described herein In this example the prechambers (1) are formed by an insert (3) each and at least one wall of the inclined channel gravity cell separator (10). Each of the two inserts (3) in this example is inserted until stop into the receiving chamber (4) of the inclined channel gravity cell separator (10) from above via vertical groves (not shown), which ensure that the inserts (3) do not contact the wall of the receiving chamber. As in this example the wall of said receiving chamber is made from electropolished steel it is advantageous that the inserts to not touch the walls, since scratching is avoided.

## Claims

1. Prechamber for an inclined channel gravity cell separator with a multitude of channels for retaining cells from a reactor mixture, wherein said prechamber comprises at least one inlet for flow distribution of an incoming fluid stream and at least one aperture towards the multitude of channels and wherein said prechamber encloses a 3-dimensional space.

2. Prechamber according to claim 1, wherein said prechamber is formed by an insert and at least one wall of the inclined channel gravity cell separator and wherein at least one opening is present opposite the at least one aperture.

3. Prechamber according to claims 1-2, wherein each of said at least one aperture consist of at least one indentation, which is open and at least one elevation, which is closed.

4. Prechamber according to claim 1, wherein said prechamber is positioned outside of the inclined channel gravity cell separator and wherein said prechamber either alone or in combination with at least one wall of the inclined channels gravity cell separator forms the at least one aperture.

5. Inclined channel gravity cell separator with a multitude of channels for retaining cells from a reactor mixture comprising
• at least one receiving chamber comprising at least one collection region for collecting the cells using gravity,
• wherein the receiving chamber has an outlet at the bottom allowing the retained cells to exit the inclined channel gravity cell separator and wherein the receiving chamber is in fluid communication with the multitude of channels which are opened at their lower end and at their upper end and
• wherein the inclined channel gravity cell separator further comprises at least one prechamber according to claims 1-4, wherein the at least one aperture of the at least one prechamber is in direct fluid communication with the channels of the inclined channel gravity cell separator.

6. Inclined channel gravity cell separator according to claim 5, wherein the inclined channel gravity cell separator comprises one receiving chamber with two prechambers, wherein each prechamber comprises one inlet and wherein said prechamber is formed by an insert and at least one wall of the inclined channel gravity cell separator and wherein at least one opening is present opposite the at least one aperture of each prechamber.

7. Use of a prechamber according to claims 1-4 or an inclined channel gravity cell separator according to claims 5-6, for cell separation.

8. Method of separating cells from a reactor mixture and retaining the cells comprising the following steps:
- providing a reactor mixture, comprising cells to be separated, to an inclined channel gravity cell separator according to claims 5-6 comprising at least one prechamber via at least one inlet
- flow distribution of the reactor mixture into the channels of the inclined channel gravity cell separator via the at least one aperture of the at least one prechamber
- separation of the cells from the reactor mixture in the channels of the inclined channel gravity cell separator wherein the clarified reactor mixture exits the channels at their top end and the separated cells slide down the channel wall,
- collection of the separated cells in the receiving chamber of the inclined channel gravity cell separator.

9. Insert for generating a prechamber according to claim 2, wherein said insert has a body allowing for the formation of at least one aperture towards the multitude of channels as well as for the formation of the at least one opening opposite the at least one aperture, when the insert is positioned in the inclined channel gravity cell separator.
